# EUROPÄISCHE PATENTSCHRIFT

(11) **EP 3 383 451 B1**
(45) Veröffentlichungstag und Bekanntmachung des Hinweises auf die Patenterteilung: **28.04.2021**
(21) Anmeldenummer: 16805294.2
(22) Anmeldetag: 02.12.2016
(51) Int. Cl.: A61M 1/28, A61M 25/00, A61J 1/10, A61M 1/36

(54) **DIALYSEGERÄT**
DIALYSIS APPARATUS
APPAREIL DE DIALYSE

(30) Priorität: 03.12.2015 DE 102015015624
(43) Veröffentlichungstag der Anmeldung: 10.10.2018
(73) Patentinhaber: Fresenius Medical Care Deutschland GmbH, 61352 Bad Homburg (DE)
(72) Erfinder: TESSENDORF, Bernd, 47608 Geldern (DE)
(74) Vertreter: Herrmann, Uwe
(86) Internationale Anmeldenummer: PCT/EP2016/002036
(87) Internationale Veröffentlichungsnummer: WO 2017/092871

(56) Entgegenhaltungen:
- DE-A1-102010 038 923
- DE-A1-102013 014 097
- US-A1- 2010 051 552
- US-A1- 2011 186 517
- US-A1- 2014 018 727

## Beschreibung

Die vorliegende Erfindung betrifft ein Dialysegerät, insbesondere ein Peritoneal-dialysegerät, mit einer oder mehreren der folgenden Komponenten: wenigstens einem Behältnis, insbesondere wenigstens einem Beutel, zur Aufnahme frischer oder gebrauchter Dialysierflüssigkeit, wenigstens einem Schlauchset zur Führung von Dialysierflüssigkeit, und/oder wenigstens einem Patientenkatheter zur Einleitung und/oder Abfuhr von Dialysierflüssigkeit in den bzw. aus dem Bauchraum eines Patienten.

Aus dem Stand der Technik bekannte Dialysegeräte werden üblicherweise basierend auf einer sogenannten Verschreibung, die seitens des Arztes vor der Behandlung festgelegt wird, betrieben. Die Behandlung erfolgt somit im Allgemeinen "statisch", d.h. wird nach einem bestimmten Protokoll durchgeführt. Dies kann dazu führen, dass die Behandlung möglicherweise nicht optimal hinsichtlich der Behandlungseffizienz bzw. des Behandlungskomforts für den Patienten ist. So sind insbesondere die Konzentration der Dialysierflüssigkeit, die Verweildauer im Bauch, die Anzahl der Behandlungszyklen, die Ein- und Auslaufgeschwindigkeiten in der Praxis vorab festgelegt und sind während der Behandlung fix, d.h. werden nach einem vorgegebenen Verschreibungsprotokoll eingestellt.

Des Weitern wird bei aus dem Stand der Technik bekannten Peritonealdialysegeräten die Dialysierflüssigkeit, die mittels des Patientenkatheters, der in den Bauchraum führt, abgelassen wird, üblicherweise nicht analysiert, sondern entsorgt. Es werden nur in unregelmäßigen Abständen Tests durchgeführt, die bspw. darauf abzielen, ob das Peritoneum noch ausreichend funktionsfähig ist. Diese Tests werden in der Regel aufwändig beim Arzt durchgeführt.

Aus der EP 0 772 693 B1 ist ein Verfahren und eine Vorrichtung zur Messung der Konzentration einer Substanz, wie bspw. Harnstoff in einer Dialyselösung bekannt. Die dazu eingesetzten Sensoren sind bspw. Leitfähigkeitssensoren. Die EP 0 711 182 B1 beschreibt ebenfalls die Bestimmung von Harnstoff im Rahmen der Dialysebehandlung, wobei die Flüssigkeitsprobe bspw. an einem Sensor vorbeigeführt wird, der Bestandteil einer Probenahmeeinheit des Dialysegerätes ist. Aus der EP 0 942 759 B1 ist ein Dialysegerät mit einer Harnstoffüberwachungseinheit bekannt, die zur Überwachung der Behandlungseffizienz und zur automatischen Anpassung der Behandlung dient.

Die WO 2013/170219 A1 offenbart ein Überwachungssystem mit einem unter der Haut des Patienten implantierten Sensors, wobei die Messdaten des Sensors optisch ausgelesen werden. Aus der EP 0 782 460 B1 ist eine Vorrichtung zur Durchführung eines sogenannten Peritonealgleichgewichtstests bekannt, bei dem eine Vielzahl an Stoffwechselabbauprodukten bestimmt wird, um auf die Transporteigenschaften des Peritoneums schließen und darüber eine patientenspezifische Peritonealdialyseverschreibung festlegen zu können.

Die WO 2015/012990 A1 beschreibt schließlich die Weiterleitung von Alarmmeldungen von einem Peritonealdialysegerät z. B. auf ein Handy.

Die DE 102010038923 A1 offenbart eine Dialyse-Kassette zur Heimhämodialyse.

Weiterhin offenbart die US 2014/0018727 A1 ein Peritonealdialysegerät mit einem Drucksensor im Kreislauf für die Peritonealflüssigkeit.

Darüber hinaus offenbart die US 2011/0186517 A1 ein Dialysegerät, welches bevorzugt mit Mehrkammerbeuteln zum Einsatz kommt. Das korrekte Mischen der verschiedenen in den Mehrkammerbeuteln enthaltenen Komponenten wird heirbei überwacht.

Zudem ist aus der DE 102013014097 A1 ein Einwegartikel zur Verwendung in der Dialysebehandlung bekannt, der einen Sensor zur Ermittlung eines die Dialyse betreffenden Messwerts aufweist.

Zusätzlichen Stand der Technik stellt die Druckschrift US 2010/0051552 A1 dar.

Der vorliegenden Erfindung liegt die Aufgabe zugrunde, ein Dialysegerät, insbesondere ein Peritonealdialysegerät der eingangs genannten Art dahingehend weiterzubilden, dass dieses auf besonders einfache Art und Weise eine Überwachung Darüber hinaus offenbart die US 2011/0186517 A1 ein Dialysegerät, welches bevorzugt mit Mehrkammerbeuteln zum Einsatz kommt. Das korrekte Mischen der verschiedenen in den Mehrkammerbeuteln enthaltenen Komponenten wird heirbei überwacht.

Zudem ist aus der DE 102013014097 A1 ein Einwegartikel zur Verwendung in der Dialysebehandlung bekannt, der einen Sensor zur Ermittlung eines die Dialyse betreffenden Messwerts aufweist.

Zusätzlichen Stand der Technik stellt die Druckschrift US 2010/0051552 A1 dar.

Der vorliegenden Erfindung liegt die Aufgabe zugrunde, ein Dialysegerät, insbesondere ein Peritonealdialysegerät der eingangs genannten Art dahingehend weiterzubilden, dass dieses auf besonders einfache Art und Weise eine Überwachung der Behandlung und ggfs. eine Anpassung von Behandlungsparametern ermöglicht.

Diese Aufgabe wird durch ein Dialysegerät mit den Merkmalen des Anspruchs 1 gelöst.

Danach ist vorgesehen, dass wenigstens eine der Komponenten, d.h. wenigstens das Behältnis und/oder das Schlauchset und/oder der Patientenkatheter zumindest einen Sensor aufweist, der ausgebildet ist, wenigstens einen Parameterwert der in der jeweiligen Komponente befindlichen Flüssigkeit zu messen und der des Weiteren ausgebildet ist, den Parameterwert an zumindest einen Empfänger zu übertragen. Der Sensor kann so ausgebildet sein, dass der Parameterwert einer ruhenden oder einer strömenden Flüssigkeit gemessen wird.

Der vorliegenden Erfindung liegt somit der Gedanke zugrunde, die Sensorik als Bestandteil der wenigstens einen Komponente vorzusehen. Der oder die Sensoren sind bspw. auf oder im Beutel bzw. auf oder im Patientenkatheter bzw. auf oder im Schlauchset angeordnet und somit kein fester Bestandteil des Dialysegerätes, was dementsprechend einfacher aufgebaut sein kann als bekannte Geräte, die eine derartige Sensorik aufweisen.

Der wesentliche Gedanke der Erfindung besteht somit darin, den wenigstens einen Sensor als Bestandteil einer oder mehreren der genannten Komponenten auszuführen.

Vorzugsweise handelt es sich bei der Komponente um einen Wegwerfartikel, d.h. um einen Disposable, so dass auch der Sensor mit verworfen wird.

Durch die vorliegenden Erfindung ist es möglich, auf einfache Art und Weise eine Überwachung der Behandlung durchzuführen, bspw. dem Patienten die Information zukommen zu lassen, ob die durchgeführte Behandlung erfolgreich abläuft oder dem Arzt die Information zukommen zu lassen, ob die Behandlung erfolgreich abgelaufen ist bzw. gerade abläuft, um ggfs. Behandlungsparameter anzupassen und z. B. eine wirksamere oder schonendere Behandlung zu verschreiben. Diese kann z. B. darin bestehen, dass die Glukosebelastung des Patienten durch die Dialyseflüssigkeit verringert wird und somit eine besser verträgliche und ggf. effizientere Therapie erzielt wird.

Dieser Eingriff bzw. die Änderung der Verschreibung kann nach einer Behandlung oder auch vorzugsweise während einer Behandlung und besonders bevorzugt in Echtzeit erfolgen. D.h. der Arzt oder auch der Patient kann mit dem Erhalt der Sensordaten erforderlichenfalls auf die Behandlung Einfluss nehmen und einen oder mehrere der Behandlungsparameter verändern.

So können bspw. zur Bewertung des Behandlungserfolges die Informationen gesammelt werden, welche Clearance-Rate erreicht wurde, d.h. wieviel Gift in der aus dem Bauchraum herausgelassenen Dialysierflüssigkeit enthalten ist, es kann eine Aussage über die Funktionsfähigkeit des Peritoneums getroffen werden, insbesondere kann eine Aussage über die frühzeitige Erkennung einer zu geringen Ultrafiltrationsrate und auch eine frühzeitige Erkennung einer Peritonitis erfolgen.

Somit ist es denkbar, eine in Echtzeit erfolgende Anpassung der Behandlungsparameter basierend auf einem oder mehreren der Senordaten vorzunehmen.

Besonders vorteilhaft ist es, wenn der Sensor ausgebildet ist, den wenigstens einen Parameterwert drahtlos an zumindest einen Empfänger zu übertragen.

Der Sensor ist erfindungsgemäß dazu ausgebildet, den mindestens einen Parameterwert kontinuierlich zu messen und/oder zu übermitteln, so dass eine durchgehende Überwachung der Behandlung bzw. eine durchgehende Aufzeichnung der Behandlung möglich ist.

Der Sensor kann so ausgebildet und angeordnet sein, dass er von der Flüssigkeit überströmt wird oder auch durchströmt wird.

Bei der Flüssigkeit handelt es sich insbesondere um die Dialysierflüssigkeit selbst.

Der wenigstens eine Sensor kann in die Wandung der Komponente, wie bspw. des Beutels, integriert sein oder auch innen oder außen an der Komponente angebracht sein. Wesentlich ist, dass der Sensor kein externer Bestandteil der Komponente ist, sondern in oder an dieser angebracht ist.

In einer weiteren Ausgestaltung der Erfindung ist vorgesehen, dass der wenigstens eine Sensor als passiver Transponder, bspw. als RFID-Chip, ausgeführt ist oder auch als aktiver Transmitter, d.h. als aktiver, mit einer Energiequelle ausgestatteter Sender bzw. als aktiver Transponder.

Die Datenübertragung vom Sensor an einen Empfänger kann auf jede beliebige Art und Weise erfolgen, auch drahtgebunden, wobei eine drahtlose Übertragung bevorzugt ist. Denkbar ist die Übertragung mittels RFID, ZigBee, bluetooth etc.

Ist der Sensor als passiver Transponder ausgeführt, d.h. verfügt dieser über keine eigene Stromversorgung, ist eine besonders kostengünstige Ausführung der fraglichen Komponente möglich.

In einer bevorzugten Ausführung der Erfindung besteht das Schlauchset aus zumindest zwei Abschnitten, die an einer Verbindungsstelle, wie z. B. an einem Brechkonnektor in Fluidverbindung stehen. Der Sensor kann an dieser Verbindungsstelle angeordnet sein und zwar derart, dass er mit den beiden voneinander getrennten Abschnitten in Verbindung steht und vorzugsweise zwischen diesen angeordnet ist.

So ist es bspw. möglich, bei einer Peritonealdialyse den Sensor in der zum Drainagebeutel führenden Leitung anzuordnen. Dabei kann der Sensor zwischen einem ersten Schlauchstück angeordnet werden und zwischen einem zweiten Schlauchstück, das mit dem Drainagebeutel in Fluidverbindung steht, wobei es sich bei dem zweiten Schlauchstück um das Schlauchstück des Schlauchsets handeln kann, das im Rahmen der vorherigen Behandlung bzw. eines vorherigen Zyklus mit dem oder den die frische Dialyseflüssigkeit enthaltenden Behältnissen bzw. Beuteln in Verbindung stand.

Bei dem wenigstens einen Parameter, kann es sich bspw. um die Temperatur, den Druck, die Konzentration eines oder mehrerer Stoffwechselabbauprodukte (z.B. Harnstoff, Kreatinin und Elektrolyte), den Glukosegehalt, den Proteingehalt, die Trübung, die Leitfähigkeit der Flüssigkeit, d.h. insbesondere der Dialysierflüssigkeit oder auch des Blutes, handeln.

Diese Aufzählung ist nicht beschränkend sondern exemplarischer Natur. Grundsätzlich ist jeder beliebige Parameter, der durch einen Sensor erfassbar ist, von der Erfindung mit umfasst.

Das Dialysegerät kann weist erfindungsgemäß einen Empfänger auf, der geeignet ist, den oder die seitens des oder der Sensoren der Komponenten ermittelten Parameterwerte zu empfangen. Das Dialysegerät kann des Weiteren wenigstens eine Anzeigeeinheit und/oder wenigstens eine Auswerteeinheit aufweisen, wobei die Anzeigeeinheit den oder die Parameter anzeigen kann oder auch auf sonstige Weise z. B. auch akustisch ausgeben kann.

Die Auswerteeinheit ist erfindungsgemäß dazu ausgebildet, den Betrieb des Dialysegerätes in Abhängigkeit des oder der Parameterwerte zu steuern oder zu regeln.

Dabei ist vorzugsweise vorgesehen, dass diese Steuerung oder Regelung des Dialysegerätes in Echtzeit erfolgt.

Die Auswertung der Daten z. B. der Anteil der Gifte bzw. Stoffwechselabbauprodukte der im Drainagebeutel eines Peritonaldialysegerätes enthaltenen Flüssigkeit ermöglicht bspw. eine Indikation an den Arzt, der ggfs. genauere Folgeuntersuchungen einleiten kann und/oder eine Echtzeit-Anpassung der Peritonealdialyseparameter, wie bspw. der Verweildauer der Flüssigkeit im Bauchraum etc. Dies ermöglicht eine optimale, Echtzeit-geregelte Peritonealdialysebehandlung.

Wie bereits oben ausgeführt, handelt es sich bei der oder den Komponenten vorzugsweise um Wegwerfartikel, so dass auch die Sensoren nach einer Patientenbehandlung verworfen werden. Dadurch ergibt sich eine stets sterile Handhabung.

Das Dialysegerät kann des Weiteren derart ausgebildet sein, dass dessen Betrieb nicht nur in Abhängigkeit von den Daten gesteuert oder geregelt wird, die von dem oder den Sensoren der Komponente(n) stammen, sondern auch von externen Sensoren, d.h. von Sensoren, die nicht Bestandteil der fraglichen Komponenten (Beutel bzw. Behälter, Schlauchset, Katheter) sind. Somit kann die Behandlung auch unter Berücksichtigung der Daten anderer Sensoren, wie bspw. der Daten erfolgen, die von einem Mobiltelefon oder von körpergetragenen Sensoren stammen. Die Daten können auch von externen Geräten, wie beispielsweise von Waagen oder auch Blutdruckmessgeräten etc. stammen.

Somit kann die Behandlung in Abhängigkeit z. B. auch von Bioparametern, wie bspw. der Herzrate oder Körpertemperatur oder dergleichen gesteuert oder geregelt werden.

Der oder die Empfänger, an die die Daten des oder der Sensoren übermittelt werden, sind vorzugsweise Bestandteile des Dialysegerätes.

In einer weiteren Ausgestaltung der Erfindung ist vorgesehen, dass es sich bei dem oder den Empfängern nicht um einen Bestandteil des Dialysegerätes handelt, sondern um wenigstens ein externes Gerät, vorzugsweise um ein mobiles Endgerät wie bspw. um ein Smartphone oder Tablet.

So ist es möglich, die mittels des oder der Sensoren gemessenen Daten vorzugsweise kontinuierlich aufzuzeichnen und auf ein Handy oder Tablet zu übertragen. Diese Übertragung erfolgt vorzugsweise drahtlos. Auch die Übertragung auf den Computer des Arztes ist denkbar.

Vorzugsweise werden die Daten dabei derart aufbereitet, dass sie dem Patienten bzw. Anwender bzw. Arzt auf einer App zur Verfügung gestellt werden können, um ihn über den aktuellen Behandlungserfolg zu informieren. Auf dem fraglichen Gerät, das den Empfänger darstellt kann somit eine App installiert sein, die ausgebildet ist, dass dem Nutzer des Gerätes der oder die Parameterwerte angezeigt werden. Auch ist es denkbar, dass zwischen dem Gerät, das den Empfänger darstellt, und dem Dialysegerät eine Kommunikationsverbindung besteht, die geeignet ist, dass der Nutzer des Gerätes das Dialysegerät und damit auch die Behandlung steuern oder regeln kann.

Wie bereits oben ausgeführt, ist es anhand der erfassten Daten weiterhin möglich, die Verschreibung einer Behandlung vorzugsweise in Echtzeit, d.h. während der Behandlung anzupassen.

Weiterhin betrifft die Erfindung ein Kommunikationssystem umfassend wenigstens ein Dialysegerät gemäß einem der Ansprüche 1 und 8 und umfassend wenigstens einen Empfänger, der ausgebildet ist, den wenigstens einen Parameterwert zu empfangen, wobei es sich bei dem Empfänger nicht um einen Bestandteil des Dialysegerätes, sondern um ein externes Gerät, insbesondere um ein mobiles Gerät und besonders bevorzugt um ein Handy oder ein Tablet, handelt.

Die Offenbarung umfasst des Weiteren eine Komponente, ausgewählt aus den folgenden Gruppe: Behältnis,vorzugsweise Beutel, zur Aufnahme frischer oder gebrauchter Dialysierflüssigkeit, Schlauchset zur Führung einer Flüssigkeit, Patientenkatheter zur Einleitung und/oder Abfuhr von Dialysierflüssigkeit in den bzw. aus dem Bauchraum eines Patienten. Die wenigstens eine der genannten Komponenten weist zumindest einen Sensor auf, der ausgebildet ist wenigstens einen Parameterwert der Flüssigkeit zu messen und der des Weiteren ausgebildet ist, diesen Parameterwert an zumindest einen Empfänger zu übertragen. Die genannte Komponente ist vorzugsweise mit den Merkmalen eines der Ansprüche 1 bis 8 ausgeführt.

Die Offenbarung betrifft des Weiteren die Verwendung einer derartigen Komponente in einem Dialysegerät der Erfindung.

Wie weiter oben ausgeführt, ist der Sensor Bestandteil der fraglichen Komponente. Er kann z. B. innerhalb eines Peritonealdialysebeutels, im Einlauf- oder im Auslaufbeutel oder in beiden angeordnet sein. Dabei können die Beutel so ausgebildet sein, dass der Sensor in deren Wandung integriert ist. Dies gilt für die weiteren Komponenten (Schlauchset, Katheter) entsprechend. Auch die Anordnung des Sensors außerhalb der Komponenten oder auch innerhalb der Komponenten ist denkbar und von der Erfindung mit umfasst.

Wie oben ausgeführt, ist es denkbar, dass es sich bei dem Sensor um einen aktiven oder passiven Transponder bzw. Transmitter handelt. Besonders bevorzugt ist es, wenn der Sensor keine eigene Energieversorgung aufweist, weil die fragliche Komponente dann besonders günstig hergestellt werden kann. Denkbar ist es weiterhin, dass der Sensor so angeordnet ist, dass er im Bereich einer strömenden Flüssigkeit angeordnet ist und dass die zum Betrieb des Sensors erforderliche Energie z. B. über ein Propellerrädchen oder dergleichen aus dem strömenden Fluid bezogen wird.

Die vorliegende Erfindung betrifft sowohl das Dialysegerät bzw. dessen Komponenten im mit Flüssigkeit gefüllten Zustand als auch in dem Zustand, in dem sich keine Flüssigkeit darin befindet.

Weitere Einzelheiten und Vorteile werden anhand eines in der Zeichnung dargestellten Ausführungsbeispiels näher erläutert. Es zeigen:

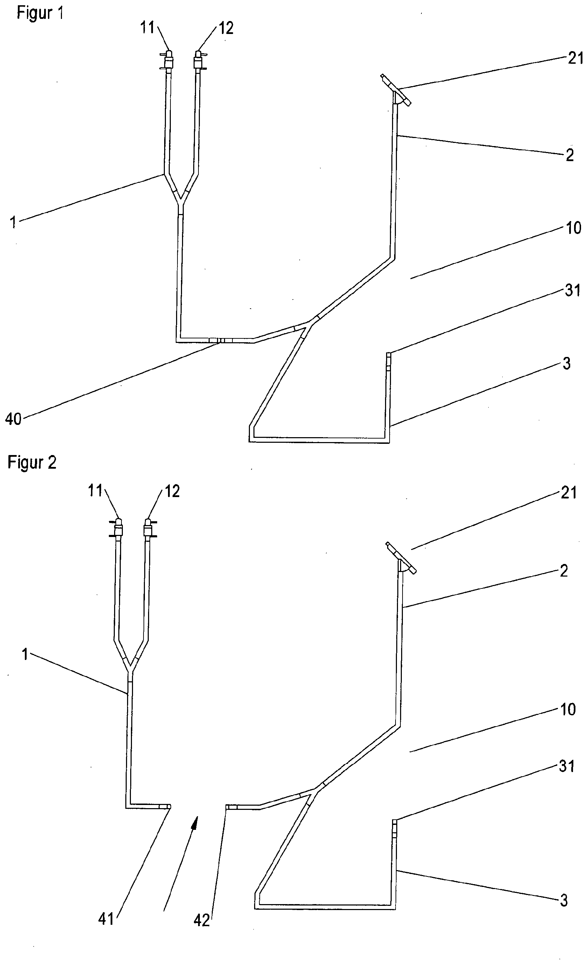

- Fig. 1- 3:: Darstellung eines Schlauchsets vor und nach dem Einsatz eines Sensors gemäß der Erfindung.

In Figur 1 ist mit dem Bezugszeichen 10 ein Schlauchset dargestellt, dass zum Einsatz in der Peritonealdialyse bestimmt ist. Das Schlauchset weist einen ersten Abschnitt 1 auf, der über Konnektoren 11, 12 mit Beuteln verbindbar ist, die frische Dialysierflüssigkeit enthalten.

Das Schlauchset weist des Weiteren einen Abschnitt 2 auf, der über das Anschlussstück 21 mit dem Patientenkatheter, d.h. mit dem Katheter in Verbindung steht, der in den Bauchraum des Patienten führt.

Das Schlauchset weist des Weiteren einen dritten Abschnitt 3 auf, der bei der Entleerung des Bauchraums von gebrauchter Dialysierflüssigkeit mittels des Konnektors 31 mit einem Drainagebeutel verbunden wird.

Das Bezugszeichen 40 kennzeichnet einen Brechkonnektor, z. B. einen Luer-Locckonektor, mittels dessen der Abschnitt 1 des Schlauchsets in zwei Teile auftrennbar ist, deren Enden bzw. Konnektoren mit den Bezugszeichen 41, 42 gekennzeichnet sind.

Figur 1 zeigt das Schlauchset 10 in dem Zustand, in dem aus den nicht dargestellten Beuteln, enthaltend die frische Dialysierflüssigkeit, diese über den Abschnitt 1 und 2 und über den nicht dargestellten Patientenkatheter in den Bauchraum des Patienten eingeleitet wird.

Im Anschluss an dieses Einleiten und nach Ablauf einer bestimmten Verweilzeit im Bauchraum des Patienten kann das Schlauchset an der Stelle 40 aufgetrennt werden, wie dies in Figur 2 durch einen Pfeil dargestellt ist.

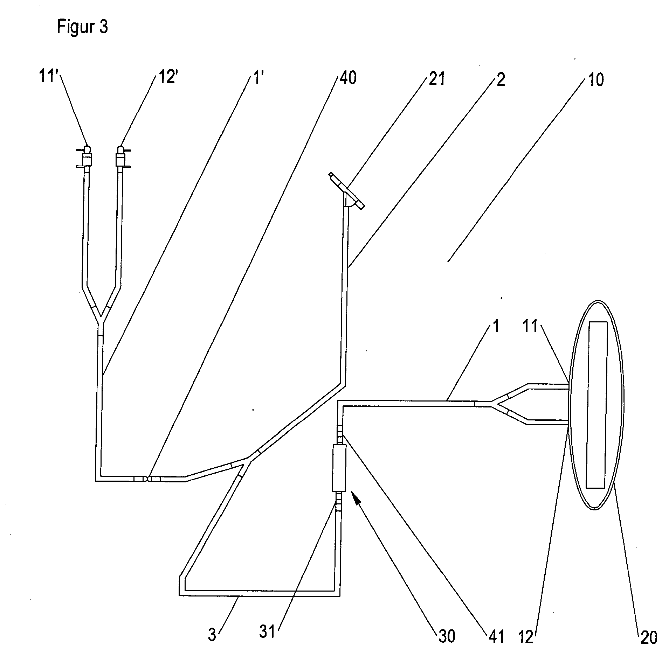

Zum Entleeren der Dialyseflüssigkeit aus dem Bauchraum wird der benutzte Schlauchsetabschnitt 1, der von den Konnektoren 11, 12 zu dem Konnektor 41 reicht, gemäß Figur 3 mit dem Drainagebeutel 20 und des Weiteren mit dem Konnektor 31 des Abschnitts 3 des Schlauchsets verbunden. Des Weiteren wird ein neues Schlauchset 1' bereitgestellt, das mit dem Konnektor 42 verbunden wird und das mittels der Konnektoren 11' und 12' mit Beuteln verbunden wird, die frische Dialysierflüssigkeit enthalten.

Wie dies aus Figur 3 hervorgeht, befindet sich zwischen dem Ende (Konnektor 31) des Abschnittes 3 und dem Ende (Konnektor 41) des benutzten Abschnitts 1 der Sensor 30. Dieser ist somit aufgrund seiner Anordnung und als Bestandteil des Schlauchsets in der Lage, z. B. die Zusammensetzung, die Stoffwechselprodukte (z.B. Harnstoff, Kreatinin und Elektrolyte), die Leitfähigkeit, die Temperatur oder einen sonstigen beliebigen Parameter der aus dem Bauchraum ablaufenden Dialysierflüssigkeit zu erfassen. Das Ergebnis dieser Messung kann bspw. in Echtzeit dem Patienten und/oder auch dem Arzt zur Verfügung gestellt werden. Grundsätzlich kann der Sensor 30 auch an anderer Stelle z.B. am Schlauch oder auch am Patienten-Konnektor 21 angeordnet sein.

Basierend auf diesen Messungen kann die Behandlung bzw. die Verschreibung des Patienten, wie z. B. die Konzentration gewisser Substanzen in der frischen Dialysierflüssigkeit oder die Verweildauer im Bauchraum entsprechend optimal angepasst werden. Es kann somit eine auf den Patienten zugeschnittene Behandlung durchgeführt werden.

Von der Erfindung ist jedoch auch der Fall umfasst, dass auf die laufende Behandlung kein Einfluss genommen wird, sondern dass die Sensordaten gesammelt werden und dann nach der Behandlung des Patienten einer Auswertung zugeführt werden.

Unabhängig davon, ob eine Echtzeitsteuerung oder -regelung der Behandlung erfolgt, kann das Dialysegerät wenigstens einen Speicher aufweisen, in dem die von dem oder den Sensoren erfassten Parameterwerte auslesbar gespeichert werden.

Besonders vorteilhaft ist es, wenn der Sensor über einen Sender verfügt, der in der Lage ist, drahtlos den oder die Parameterwerte z. B. auf ein mobiles Endgerät des Patienten zu übersenden. Der Patient kann bspw. über ein Handy verfügen oder über ein Tablet oder dergleichen oder auch über einen Computer, der über eine entsprechende Schnittstelle zum Empfang der Daten verfügt. Auf diesen Geräten kann der Patient oder auch der Arzt dann die entsprechenden Parameterwerte ansehen und ggfs. bewerten.

## Patentansprüche

1. Peritonealdialysegerät, aufweisend die folgenden Komponenten: wenigstens ein Behältnis, vorzugsweise Beutel (20), zur Aufnahme frischer oder gebrauchter Dialysierflüssigkeit, wenigstens ein Schlauchset (10) zur Führung von Dialysierflüssigkeit, und wenigstens ein Patientenkathether zur Einleitung und/oder Abfuhr von Dialysierflüssigkeit in den bzw. aus dem Bauchraum eines Patienten, wobei wenigstens eine der Komponenten zumindest einen Sensor (30) aufweist, der ausgebildet ist, wenigstens einen Parameterwert der in der Komponente befindlichen Flüssigkeit zu messen, und der des Weiteren ausgebildet ist, den gemessenen Parameterwert an zumindest einen Empfänger zu übertragen
wobei das Peritonealdialysegerät wenigstens einen Empfänger aufweist, der ausgebildet ist, den oder die seitens des oder der Sensoren ermittelten Parameterwerte zu empfangen und dass das Peritonealdialysegerät wengistens eine Auswerteeinheit aufweist, wobei die Auswerteeinheit ausgebildet ist, den Betrieb des Peritonealdialysegerätes in Abhängigkeit des oder der Parameterwerte zu steuern oder zu regeln, wobei die Auswerteineinheit ausgebildet ist, den Betrieb des Dialysegerätes in Abhängigkeit von Parameterwerten zu steuern oder zu regeln, die von einem oder mehreren weiteren Sensoren stammen, die nicht mit der bzw. den Komponenten in Verbindung stehen,
**dadurch gekennzeichnet dass** der Sensor ausgebildet ist, den wenigstens einen Parameterwert der in der Komponente befindlichen Flüssigkeit drahtlos und kontinuierlich an zumindest einen Empfänger zu übertragen, wobei es sich bei dem Empfänger nicht um einen Bestandteil des Peritonealdialysegerätes handelt, sondern um ein externes, vorzugsweise um ein mobiles, Endgerät, wie ein Smartphone oder Tablet.

2. Peritonealdialysegerät nach Anspruch 1, **dadurch gekennzeichnet, dass** der Sensor derart ausgebildet ist, dass dieser von der Flüssigkeit um- oder durchströmt wird.

3. Peritonealdialysegerät nach einem der vorhergehenden Ansprüche, **dadurch gekennzeichnet, dass** der wengistens eine Sensor in die Wandung der Komponente integriert ist oder innen oder außen an der Komponente angebracht ist.

4. Peritonealdialysegerät nach einem der vorhergehenden Ansprüche, **dadurch gekennzeichnet, dass** der wengistens eine Sensor als passiver Transponder oder als aktiver Transmitter ausgebildet ist.

5. Peritonealdialysegerät nach einem der vorhergehenden Ansprüche, **dadurch gekennzeichnet, dass** das Schlauchset aus wenigstens zwei Abschnitten besteht, die an einer Verbindungsstelle in Fluidverbindung stehen und dass der Sensor an dieser Verbindungsstelle angeordnet ist, so dass der Sensor mit beiden Abschnitten in Verbindung steht und vorzugsweise zwischen diesen angeordnet ist.

6. Peritonealdialysegerät nach einem der vorhergehenden Ansprüche, **dadurch gekennzeichnet, dass** es sich bei dem Parameter um die Temperatur, um den Druck, um die Konzentration eines oder mehrere Stoffwechselabbauprodukte, um den Glucosegehalt, um den Proteingehalt, um die Trübung und/oder um die Leitfähigkeit der Flüssigkeit handelt.

7. Peritonealdialysegerät nach einem der vorhergehenden Ansprüche, **dadurch gekennzeichnet, dass** das Peritonealdialysegerät wengistens eine Anzeigeeinheit aufweist, wobei die Anzeigeeinheit ausgebildet ist, den oder die Parameterwerte anzuzeigen oder auf sonstige Weise auszugeben, wobei vorzugsweise vorgesehen ist, dass die Auswerteeinheit derart ausgebildet ist, dass die Steuerung oder Regelung des Dialysegerätes in Echtzeit erfolgt.

8. Peritonealdialysegerät nach einem der vorhergehenden Ansprüche, **dadurch gekennzeichnet, dass** es sich bei der Komponente um ein Disposable handelt.

9. Kommunikationssystem umfassend wengistens ein Peritonealdialysegerät gemäß einem der Ansprüche 1 bis 8 und umfassend wenigstens einen Empfänger, der ausgebildet ist, den Parameterwert zu empfangen, wobei es sich bei dem Empfänger nicht um einen Bestandteil des Dialysegerätes, sondern um ein dazu extern angeordnetes Gerät, insbesondere um ein mobiles Endgerät, wie z.B. um ein Smartphone oder um ein Tablett handelt.

## Claims

1. Peritoneal dialysis machine, comprising the following components: at least one receptacle, preferably a bag (20) to receive fresh or used dialysate, at least one tubing set (10) for conducting dialysate, and at least one patient catheter for the inflow and/or outflow of dialysate into or from the patient's abdominal cavity, wherein at least one of the components comprises at least one sensor (30) which is configured for measuring at least one parameter value of the fluid located inside the component, and which is also configured to transmit the measured parameter value to at least one receiver,
wherein the peritoneal dialysis machine comprises at least one receiver which is configured to receive the one or more parameter values determined by the one or more sensors, and the peritoneal dialysis machine comprises at least one evaluation unit, wherein the evaluation unit is configured to control or regulate the operation of the peritoneal dialysis machine depending on the one or more parameter values, wherein the evaluation unit is configured to control or regulate the operation of the dialysis machine depending on parameter values originating from one or more further sensors not in communication with the one or more components,
**characterized in that**
the sensor is configured to transmit the at least one parameter value of the fluid located in the component wirelessly and continuously to at least one receiver, wherein the receiver is not part of the dialysis machine, but an external terminal device, in particular a mobile terminal device such as a smartphone or tablet.

2. Peritoneal dialysis machine according to Claim 1, **characterized in that** the sensor is configured such that the fluid flows around it or through it.

3. Peritoneal dialysis machine according to one of the preceding claims, **characterized in that** the at least one sensor is integrated into the wall of the component or attached inside or outside on the component.

4. Peritoneal dialysis machine according to one of the preceding claims, **characterized in that** the at least one sensor is configured as a passive transponder or as an active transmitter.

5. Peritoneal dialysis machine according to one of the preceding claims, **characterized in that** the tubing set consists of at least two sections which are in fluid connection at a connector point, and that the sensor is provided at that connector point such that the sensor is in connection with both sections and is preferably arranged between these sections.

6. Peritoneal dialysis machine according to one of the preceding claims, **characterized in that** the parameter is one of temperature, pressure, concentration of one or more metabolic breakdown products, of glucose level, protein level, turbidity and/or conductivity of the fluid.

7. Peritoneal dialysis machine according to one of the preceding claims, **characterized in that** the peritoneal dialysis machine comprises at least one display unit, wherein the display unit is configured to display or otherwise output the one or more parameter values, wherein it is preferably provided that the control or regulation of the dialysis machine occurs in real time.

8. Peritoneal dialysis machine according to one of the preceding claims, **characterized in that** the component is a disposable.

9. Communication system, comprising at least one peritoneal dialysis machine according to one of claims 1 to 8, and comprising at least one receiver which is configured to receive the parameter value, wherein the receiver is not part of the dialysis machine, but a device externally arranged for that purpose, in particular a mobile terminal device such as a smartphone or tablet.

## Revendications

1. Appareil de dialyse péritonéale, présentant les composants suivants : au moins un récipient, de préférence une poche (20), pour recevoir du liquide de dialyse frais ou usagé, au moins un ensemble de tuyaux flexibles (10) pour guider du liquide de dialyse, et au moins un cathéter de patient pour introduire et/ou évacuer du liquide de dialyse dans ou hors de la cavité abdominale d'un patient, dans lequel au moins un des composants présente au moins un capteur (30) qui est conçu pour mesurer au moins une valeur de paramètre du liquide se trouvant dans le composant et qui est conçu par ailleurs pour transmettre la valeur de paramètre mesurée à au moins un récepteur,
dans lequel l'appareil de dialyse péritonéale présente au moins un récepteur qui est conçu pour recevoir la ou les valeurs de paramètre déterminées du côté du ou des capteurs, et que l'appareil de dialyse péritonéale présente au moins une unité d'évaluation, dans lequel l'unité d'évaluation est conçue pour commander ou pour réguler le fonctionnement de l'appareil de dialyse péritonéale en fonction de la ou des valeurs de paramètre, dans lequel l'unité d'évaluation est conçue pour commander ou pour réguler le fonctionnement de l'appareil de dialyse en fonction de valeurs de paramètre, qui proviennent d'un ou de plusieurs autres capteurs qui ne sont pas reliés au ou aux composants, **caractérisé en ce que**
le capteur est conçu pour transmettre l'au moins une valeur de paramètre du liquide se trouvant dans le composant sans fil et en continu à au moins un récepteur, dans lequel le récepteur ne fait pas partie intégrante de l'appareil de dialyse péritonéale mais est un terminal externe, de préférence un terminal mobile, tel qu'un téléphone intelligent ou une tablette.

2. Appareil de dialyse péritonéale selon la revendication 1, **caractérisé en ce que** le capteur est conçu de telle manière que celui-ci est contourné ou traversé par le liquide.

3. Appareil de dialyse péritonéale selon l'une quelconque des revendications précédentes, **caractérisé en ce que** l'au moins un capteur est intégré dans la paroi du composant ou est installé à l'intérieur ou à l'extérieur sur le composant.

4. Appareil de dialyse péritonéale selon l'une quelconque des revendications précédentes, **caractérisé en ce que** l'au moins un capteur est conçu en tant que transpondeur passif ou en tant que transmetteur actif.

5. Appareil de dialyse péritonéale selon l'une quelconque des revendications précédentes, **caractérisé en ce que** l'ensemble de tuyaux flexibles consiste en au moins deux sections, qui sont en communication fluidique sur un point de liaison, et que le capteur est disposé sur ledit point de liaison de sorte que le capteur est en communication avec les deux sections et est disposé de préférence entre celles-ci.

6. Appareil de dialyse péritonéale selon l'une quelconque des revendications précédentes, **caractérisé en ce que** le paramètre est la température, la pression, la concentration d'un ou de plusieurs produits de décomposition métaboliques, la teneur en glucose, la teneur en protéines, la turbidité et/ou la conductivité du liquide.

7. Appareil de dialyse péritonéale selon l'une quelconque des revendications précédentes, **caractérisé en ce que** l'appareil de dialyse péritonéale présente au moins une unité d'affichage, dans lequel l'unité d'affichage est conçue pour afficher la ou les valeurs de paramètre ou les émettre d'une quelconque manière, dans lequel il est prévu de préférence que l'unité d'évaluation soit conçue de telle manière que la commande ou la régulation de l'appareil de dialyse a lieu en temps réel.

8. Appareil de dialyse péritonéale selon l'une quelconque des revendications précédentes, **caractérisé en ce que** le composant est un produit jetable.

9. Système de communication comprenant au moins un appareil de dialyse péritonéale selon l'une quelconque des revendications 1 à 8 et comprenant au moins un récepteur qui est conçu pour recevoir la valeur de paramètre, dans lequel le récepteur ne fait pas partie intégrante de l'appareil de dialyse mais est un appareil disposé de manière externe par rapport à celui-ci, en particulier un terminal mobile, tel qu'un téléphone intelligent ou une tablette.
